Europäisches Patentamt

European Patent Office

Office européen des brevets

(11) Publication number: 0 290 118
A2

# EUROPEAN PATENT APPLICATION

(21) Application number: 88301897.0

(22) Date of filing: 04.03.88

(51) Int. Cl.4: **C12N 9/48 , C12N 9/64 , C12N 9/68 , C12N 9/72 , C07K 13/00 , A61K 37/54**

The title of the invention has been amended (Guidelines for Examination in the EPO, A-III, 7.3).

(30) Priority: 07.03.87 GB 8705377

(43) Date of publication of application: 09.11.88 Bulletin 88/45

(84) Designated Contracting States: BE CH DE FR GB IT LI NL

(71) Applicant: BEECHAM GROUP PLC
Beecham House Great West Road
Brentford Middlesex TW8 9BD(GB)

(72) Inventor: Browne, Michael Joseph Beecham
Pharmaceuticals
Great Burgh Yew Tree Bottom Road
Epsom Surrey KT18 5XQ(GB)

(74) Representative: Valentine, Jill Barbara et al
Beecham Pharmaceuticals Patents & Trade
Marks Dept. Great Burgh Yew Tree Bottom
Road
Epsom Surrey KT18 5XQ(GB)

(54) Fibrinolytically active enzyme.

(57) A fibrinolytically active hybrid enzyme which comprises the A chain of plasmin linked to the B chain of a plasminogen activator selected from tissue-type plasminogen activator and urokinase plasminogen activator, characterised in that said chains are linked by peptide bonds via an amino acid sequence comprising the cleavage site of plasminogen.

EP 0 290 118 A2

## Novel Compounds

The present invention relates to novel hybrid enzymes, their preparation, pharmaceutical compositions containing them and their use in the treatment of thrombotic disease.

EP-0155387 discloses fibrinolytically active hybrid protein which comprises one chain of a 2-chain protease linked to a chain of a different 2-chain protease, or to the same chain of the same protease, at least one of the chains in the hybrid protein being derived from a fibrinolytically active protease, such that the hybrid protein has a catalytic site essential for fibrinolytic activity which is optionally blocked by a removable blocking group.

Examples of the hybrid protein include plasmin A-chain linked to tissue-type plasminogen activator (t-PA) B-chain or urokinase B-chain.

The hybrid proteins may be prepared by mixing a chain of one protease with a chain of another protease, optionally with dialysis, under oxidative conditions.

Alternatively, the hybrid proteins may be prepared by taking the genetic information (DNA sequence) of each protein, cutting and ligating this to construct a new DNA sequence coding for the hybrid protein, and expressing this DNA in prokaryote or eukaryote hosts.

The sequence of amino acids making up the plasminogen activator enzymes t-PA and urokinase and the nucleotide sequence for the cDNA which codes for the enzymes are known (see Pennica et al, 1983; Nature, 301, 214 and EP-0092182-A).

Plasmin is a two-chain serine protease which may be obtained by the cleavage of the single chain precursor, plasminogen, at a specific internal peptide bond. The amino acid sequence of human plasminogen is known (Wiman and Walters (1975) Eur.J. Biochem. 50, 489-494 and 58, 539-547; Wiman (1977) Eur. J. Biochem. 76, 129-137; Sottrup-Jensen et al. (1978) Fibrinolysis and Thrombolysis Vol. 3, 191-209, Raven Press, New York; and Sottrup-Jensen et al. (1978) Atlas of Protein Sequence and Structure Vol. 5, Suppl. 3, p91, National Biomedical Research Foundation, Silver Spring, MD). A partial nucleotide sequence coding for amino acid residues 272-790 of human plasminogen has also been described (Malinowski, D.P. et al., 1984, Biochemistry, 23, 4243-4250). The cleavage site of human plasminogen is located between residues arg-560 and val-561 (according to the sequence numbering of Sottrup-Jensen et al. (1978) Atlas of Protein Sequence (op.cit.)). Two species of plasminogen have been identified ( F.J. Castellino, Chemical Reviews Vol. 81 p431 (1981)): $Glu_1$ which has an N-terminal glutamic acid residue at position 1 and $lys_{77}$ which has an N-terminal lysine residue at position 77. References to plasminogen herein are understood to include both these species.

Recently a complete nucleotide sequence has been described (Forsgren, M., et al., 1987, FEBS Letters 213, 254-260). The nucleotide sequence predicts the existence of an extra, previously unreported, isoleucine residue near the N-terminus of the A-chain. This finding has been independently confirmed (McLean, J.N., et al., 1987, Nature 300, 132-137). Accordingly all sequence numbering below takes the extra isoleucine into account. In this numbering sequence the plasminogen cleavage site is located between residues arg-561 and val-562.

A novel class of fibrinolytically active hybrid proteins have now been discovered.

According to the present invention there is provided a fibrinolytically active hybrid enzyme which comprises the A chain of plasmin linked to the B chain of a plasminogen activator selected from tissue-type plasminogen activator and urokinase plasminogen activator, characterised in that said chains are linked by peptide bonds via an amino acid sequence comprising the cleavage site of plasminogen.

It will be understood that by the term "B-chain" is meant at least that portion of the B-chain containing the functional active centre of the plasminogen activator.

As used herein, the terms tissue-type and urokinase plasminogen activator denote a plasminogen activator of the group having the immunological properties defined for t-PA or u-PA, respectively, at the XXVIII Meeting of the International Committee on Thrombosis and Haemostasis, Bergamo, Italy, 27 July 1982.

It will also be understood that the terms t-PA and urokinase also include muteins thereof, for example as described in WO 86/01538 or EP-01 99574.

In a preferred aspect, the linking sequence comprises the amino acid residues 562 to 566 of plasminogen, more preferably residues 562 to 572.

In another preferred aspect the plasminogen activator is t-PA.

When the plasminogen activator is t-PA, the linking sequence may optionally also comprise the cleavage site of t-PA, in particular the amino acid residues 273 to 275 of t-PA.

The hybrid enzyme of the invention may be derivatised to provide pharmaceutically useful conjugates

2

analogous to known plasminogen activator-containing conjugates, for example:

(a) an enzyme-protein conjugate as disclosed in EP-A-O 155 388, in which the catalytic site on the enzyme which is responsible for fibrinolytic activity is blocked by a human protein attached thereto by way of a reversible linking group;

(b) an enzyme-protein conjugate as disclosed in EP-A-O 152,736, comprising at least one optionally blocked fibrinolytic enzyme linked by way of a site other than the catalytic site responsible for fibrinolytic activity to at least one human protein;

(c) a protein-polymer conjugate as disclosed in EP-A-0183503 comprising a pharmaceutically useful protein linked to at least one water soluble polymer by means of a reversible linking group; or

(d) an enzyme conjugate as disclosed in EP-A-0184363 comprising a plurality of fibrinolytic enzymes linked together through the active centres thereof by means of a removable blocking group.

The hybrid enzyme of the invention may take the place of native plasminogen activator as the enzyme or (human) protein component, as appropriate, of any of the conjugates described above.

The hybrid enzyme of the invention or. conjugate thereof can be further derivatised such that any catalytic site essential for fibrinolytic activity is blocked by a removable blocking group.

The above mentioned derivatives of the hybrid enzyme may be used in any of the methods and compositions described hereinafter for the hybrid enzyme itself.

As used herein the expression 'removable blocking group' includes groups which are removable by hydrolysis at a rate such that the pseudo-first order rate constant for hydrolysis is in the range of $10^{-6}$ sec$^{-1}$ to $10^{-2}$ sec$^{-1}$ in isotonic aqueous media at pH 7.4 at 37°C.

Such blocking groups are described in European Patent No.0009879 and include acyl groups such as optionally substituted benzoyl or optionally substituted acryloyl.

Suitable optional substituents for benzoyl blocking groups include halogen, $C_{1-6}$ alkyl, $C_{1-6}$ alkoxy, $C_{1-6}$ alkanoyloxy, $C_{1-6}$ alkanoylamino, amino, dimethylamino or p-guanidino.

Suitable optional substituents for acryloyl blocking groups include $C_{1-6}$ alkyl, furyl, phenyl or $C_{1-6}$ alkylphenyl.

In a further aspect, the invention provides a process for preparing a hybrid enzyme according to the invention which process comprises expressing DNA encoding said hybrid enzyme in a recombinant host cell and recovering the enzyme product.

The construction of the DNA encoding the hybrid enzyme can be carried out by conventional genetic engineering techniques and the DNA expressed in a prokaryote or eukaryote host, preferably a eukaryote host.

The DNA polymer comprising a nucleotide sequence that encodes the hybrid enzyme also forms part of the invention.

The process of the invention may be performed by conventional recombinant techniques such as described in Maniatis et. al., Molecular Cloning - A Laboratory Manual; Cold Spring Harbor, 1982.

In particular, the process may comprise the steps of:

i) preparing a replicable expression vector capable, in a host cell, of expressing a DNA polymer comprising a nucleotide sequence that encodes said hybrid enzyme;

ii) transforming a host cell with said vector;

iii) culturing said transformed host cell under conditions permitting expression of said DNA polymer to produce said hybrid enzyme; and

iv) recovering said hybrid enzyme.

The invention also provides a process for preparing the DNA polymer by the condensation of appropriate mono-, di-or oligomeric nucleotide units.

The preparation may be carried out chemically, enzymatically, or by a combination of the two methods, in vitro or in vivo as appropriate. Thus, the DNA polymer may be prepared by the enzymatic ligation of appropriate DNA fragments, by conventional methods such as those described by D. M. Roberts et al in Biochemistry 1985, 24, 5090-5098. The DNA fragments may be obtained by digestion of DNA containing the required sequences of nucleotides with appropriate restriction enzymes, by chemical synthesis, by enzymatic polymerisation, or by a combination of these methods.

Digestion with restriction enzymes may be performed in an appropriate buffer at a temperature of 20°-70°C, generally in a volume of 50μl or less with 0.1-10μg DNA.

Enzymatic polymerisation of DNA may be carried out in vitro using a DNA polymerase such as DNA polymerase I (Klenow fragment) in an appropriate buffer containing the nucleoside triphosphates dATP, dCTP, dGTP and dTTP as required at a temperature of 10°-37°C, generally in a volume of 50μl or less. Enzymatic ligation of DNA fragments may be carried out using a DNA ligase such as T4 DNA ligase in an appropriate buffer at a temperature of 4°C to ambient, generally in a volume of 50μl or less. The chemical synthesis of the DNA polymer or fragments may be carried out by conventional phosphotriester, phosphite or phosphoramidite chemistry, using solid phase techniques such as those described in 'Chemical and Enzymatic Synthesis of Gene Fragments - A Laboratory Manual' (ed. H.G. Gassen and A. Lang), Verlag Chemie, Weinheim (1982), or in other scientific publications, for example M.J. Gait, H.W.D. Matthes, M. Singh, B.S. Sproat, and R.C. Titmas, Nucleic Acids Research, 1982, 10, 6243; B.S. Sproat and W. Bannwarth, Tetrahedron Letters, 1983, 24, 5771; M.D. Matteucci and M.H Caruthers, Tetrahedron Letters, 1980, 21, 719; M.D. Matteucci and M.H. Caruthers, Journal of the American Chemical Society, 1981, 103, 3185; S.P. Adams et al., Journal of the American Chemical Society,1983, 105, 661; N.D. Sinha, J. Biernat, J. McMannus, and H. Koester, Nucleic Acids Research, 1984, 12, 4539; and H.W.D. Matthes et al., EMBO Journal, 1984, 3, 801.

In the preferred embodiment, a DNA molecule comprising a nucleotide sequence encoding the plasmin A chain and the amino acid sequence comprising the cleavage site of plasminogen is obtained by the digestion of a vector containing the plasminogen gene with the restriction enzyme BstXI. The digestion results in two DNA fragments which together provide a fragment (I) which encodes residues 1 to 570 and partially encodes residues 571 and 572:

```
                              ↓ Bst XI
        5'...CCA CAT TC↓                    coding strand

        3'...GGT G
                    ↑


        pro   his   ser
        570   571   572
```

The DNA fragment (I) may be ligated to a DNA molecule comprising a DNA sequence encoding the plasminogen activator B chain directly if a suitable sticky end is present on the molecule or via an oligonucleotide linker designed to provide a BstXI sticky end.

When the plasminogen activator is t-PA, the DNA molecule encoding the t-PA B chain may conveniently be obtained by digestion of a vector containing the required portion of t-PA cDNA, such as the vector pTRE24 described in EP-A-0207589 with the restriction enzymes DdeI and XhoII. The digestion provides a DNA fragment (II) which encodes residues 274 to 527 (the C-terminal residue) of t-PA and partially encodes residues 272 and 273:

```
        DdeI
          ↓
        5' T CAG TTT ...              coding strand

        3'    C AAA ...
               ↑


              phe
              274
```

A suitable oligonucleotide linker for the ligation of fragment (I) to fragment (II) is of structure (III):

```
        5'        A AGC TTA GAT CT

        3'    TA AGT TCG AAT CTA GAA GT
```
                                                            (III)

The ligation of the DNA fragments which provide fragment (I) with the fragments (II) and (III) may be carried out sequentially or simultaneously as desired, to provide the DNA molecule expressing the hybrid enzyme.

The DNA sequence at the ligation sites of fragments (I), (II), and (III), and the consequent amino acid sequence is as follows:

```
... CCA CAT TCA AGC TTA GAT CTT CAG ...
... GGT GTA AGT TCG AAT CTA GAA GTC ...


    570 571 572  *   *   *   *  273
    Pro His Ser Ser Leu Asp Leu Gln
```

Amino acids marked * are present as a result of the incorporation of the oligonucleotide linker fragment (III). Amino acids marked 570-572 are the terminal residues of a peptide sequence comprising residues 1 to 572 of plasminogen. The amino acid marked 273 is the N-terminal residue of a peptide sequence comprising residues 273 to 527 of t-PA. It will thus be apparent that the hybrid enzyme expressed by this DNA molecule includes the cleavage sites of both plasminogen (between residues 561 and 562) and of t-PA (between residues 275 and 276).

The expression of the DNA sequence encoding the hybrid enzyme in a recombinant host cell may be carried out by means of a replicable expression vector capable, in the host cell, of expressing the DNA polymer. The expression vector is novel and also forms part of the invention.

The replicable expression vector may be prepared in accordance with the invention, by cleaving a vector compatible with the host cell to provide a linear DNA segment having an intact replicon, and ligating said linear segment with a DNA sequence encoding the hybrid enzyme.

The ligation of the linear segment and the DNA molecule or fragments thereof may be carried out simultaneously or sequentially as desired.

The choice of vector will be determined in part by the host cell, which may be prokaryotic or eukaryotic, preferably eukaryotic. Suitable vectors include plasmids, bacteriophages, cosmids and recombinant viruses.

The preparation of the replicable expression vector may be carried out conventionally with appropriate enzymes for restriction, polymerisation and ligation of the DNA, by procedures described in, for example, Maniatis et al cited above. Restriction, polymerisation and ligation may be performed as described above for the preparation of the DNA polymer.

The recombinant host cell is prepared, in accordance with the invention, by transforming a host cell with a replicable expression vector of the invention under transforming conditions. Suitable transforming conditions are conventional and are described in, for example, Maniatis et al cited above, or "DNA Cloning", D.M. Glover ed., IRL Press Ltd, 1985.

The choice of transforming conditions is determined by the host cell. Thus, a bacterial host such as E.coli may be treated with a solution of $CaCl_2$ (Cohen et al, Proc. Nat. Acad. Sci., 1973, 69, 2110) or with a solution comprising a mixture of RbCl, $MnCl_2$, potassium acetate and glycerol, and then with 3-[N-morpholino]propane-sulphonic acid, RbCl and glycerol. Mammalian cells in culture may be transformed by calcium co-precipitation of the vector DNA onto the cells.

The invention also extends to a host cell transformed with a replicable expression vector of the invention.

Culturing the transformed host cell under conditions permitting expression of the DNA polymer is carried out conventionally, as described in, for example, Maniatis et al and "DNA Cloning" cited above. Thus, preferably the cell is supplied with nutrient and cultured at a temperature below 45°C.

The expression product is recovered by conventional methods according to the host cell. Thus, where the host cell is bacterial, such as E.coli it may be lysed physically, chemically or enzymatically and the protein product isolated from the resulting lysate. Where the host cell is mammalian, the product may generally be isolated from the nutrient medium. The DNA polymer may be assembled into vectors designed for isolation of stable transformed mammalian cell lines expressing the hybrid enzyme; e.g. bovine papillomavirus vectors or vectors with amplifiable markers (DNA cloning Vol.II D.M. Glover Ed. IRL Press 1985; Kaufman, R.J. et al, Molecular and Cellular Biology 5, 1750-1759, 1985; Pavlakis G.N. and Hamer, D.H., Proceedings of the National Academy of Sciences (USA) 80, 397-401, 1983; Goeddel, D.V. et al.,European Patent Application No. 0093619, 1983).

It will be appreciated that, depending upon the host cell, the hybrid enzyme prepared in accordance with the invention may be glycosylated to varying degrees. In particular, as observed by Pohl et.al., Biochemistry, 1984, 23, 3701-3707, and F.J. Castellino, Chemical Reviews Vol. 81 p431 (1981), varying degress of glycosylation may be found in unmodified, naturally occurring t-PA and plasminogen respectively. The hybrid enzyme of the invention is understood to include such glycosylated variations.

The hybrid enzyme of the invention is suitably administered in the form of a pharmaceutical composition.

Accordingly the present invention also provides a pharmaceutical composition comprising a hybrid enzyme of the invention in combination with a pharmaceutically acceptable carrier.

The compositions according to the invention may be formulated in accordance with routine procedures as pharmaceutical compositions adapted for intravenous administration to human beings.

Typically compositions for intravenous administration are solutions of the sterile enzyme in sterile isotonic aqueous buffer. Where necessary the composition may also include a solubilising agent to keep the hybrid enzyme in solution and a local anaesthetic such as lignocaine to ease pain at the site of injection. Generally, the hybrid enzyme will be supplied in unit dosage form for example as a dry powder or water free concentrate in a hermetically sealed container such as an ampoule or sachette indicating the quantity of protein in activity units. Where the hybrid enzyme includes a removable blocking group an indication of the time within which the free protein will be liberated may be given. Where the protein is to be administered by infusion, it will be dispensed with an infusion bottle containing sterile pharmaceutical grade 'Water for Injection' or saline. Where the protein is to be administered by injection, it is dispensed with an ampoule of sterile water for injection or saline. The injectable or infusable composition will be made up by mixing the ingredients prior to administration. The quantity of material administered will depend upon the amount of fibrinolysis required and the speed with which it is required, the seriousness of the thromboembolic condition and position and size of the clot. The precise dose to be employed and mode of administration must per force in view of the nature of the complaint be decided according to the circumstances by the physician supervising treatment. However, in general, a patient being treated for a thrombus will generally receive a daily dose of from 0.01 to 10 mg/kg of body weight either by injection in for example up to five doses or by infusion.

Within the above indicated dosage range, no adverse toxicological effects are indicated with the compounds of the invention.

Accordingly, in a further aspect of the invention there is provided a method of treating thrombotic diseases, which comprises administering to the sufferer an effective non-toxic amount of hybrid enzyme of the invention.

The invention also provides a hybrid enzyme of the invention for use as an active therapeutic substance and, in particular, for use in the treatment of thrombotic diseases.

The following Examples illustrate the invention.

Methods used for Examples 1 and 2

DNA cleavage

In general the cleavage of about 1µg of plasmid DNA or DNA fragments is effected using about 5 units of a restriction enzyme (or enzymes) in about 20µl of an appropriate buffer solution.

Ligation of DNA Fragments: Ligation reactions are carried out as described (Maniatis et al Molecular Cloning - A Laboratory Manual, Cold Spring Harbor Laboratory 1982). The concentration of DNA restriction fragments is estimated visually from gels on which DNA standards are also run.

Transformation of plasmid DNA into E. coli DH5α and HB101 cells is as described (Maniatis et al Molecular Cloning - A Laboratory Manual, Cold Spring Harbor Laboratory 1982).

Plasmid preparation: Large scale preparation of plasmid DNA and plasmid mini-preparations are carried out as described in Maniatis et al (Molecular Cloning - A Laboratory Manual, Cold Spring Harbor Laboratory, 1982).

Isolation of DNA fragments from low-melting-point (LMP) agarose gels

DNA fragments are isolated from LMP agarose gels as described by Maniatis et al (Molecular Cloning, A Laboratory Manual, 1982, Cold Spring Harbor).

Synthesis of oligonucleotide linkers

Synthetic oligonucleotides are made on an Applied Biosystems 381A DNA synthesizer, according to the Manufacturer's instructions.

Ligation of Synthetic Linkers to DNA

Synthetic linkers are kinased and ligated to DNA as described by Maniatis et al (Molecular Cloning, A Laboratory Manual, 1982, Cold Spring Harbor).

Detection of hybrid expression

Cell preparation: cells are trypsinised and plated out at $10^6$ cells per 60mm dish and left overnight in growth medium (10% Serum, 1% stock solution of penicillin/streptomycin; Flow Laboratories, 1% Glutamine, 1% stock solution of non-essential amino acids; Flow Laboratories, in Eagles MEM) at 37°C in a humidified incubator in an atmosphere of 5% $CO_2$/95% air.

Transfection procedure: The transfection method used (calcium coprecipitation) is described in 'DNA Cloning' Ed. D.M. Glover (Chap. 15, C. Gorman). Glycerol shock and 10mM butyrate treatment are used.

Overlay: Agarose (Indubiose $A_{37}$), 2.4g in 95ml Eagles MEM (Gibco) heated to melting point in a bunsen flame, is then placed in a water bath maintained at 48°C. 5.6 ml of fibrinogen (20mg/ml) are diluted 1:1 with 5.6 ml of Eagles MEM (containing an extra 7 mg/ml of NaCl) and retained on ice. 3.3 ml of Eagles MEM (no additions) are aliquoted into a bijou containing 86μl of bovine thrombin at 50NIH Units/ml (retained on ice). The cells are washed 3 times with Eagles MEM to remove serum. The thrombin and fibrinogen are warmed to 37°C in a water bath. 9.5 ml agarose are aliquoted into a pre-warmed universal. The thrombin is added to the agar, followed by the fibrinogen. The gel is poured over the cell layer and incubated at 37°C until lysis zones appear.

Harvesting: As an alternative to the overlay, cells are incubated in serum-free medium for 24h and the medium collected and analysed by zymography.

Nucleotide numbering throughout corresponds to that of

(i) Forsgren et al (1987) FEBS Letters 213, 254 for plasminogen-indicated by a 'p' superscript.

(ii) Pennica, D. et al (1983) Nature 301 p214 for t-PA - indicated by a 't' superscript.

Example 1

1. Construction of Plasminogen: t-PA hybrid cDNA molecules

1.1 Construction of an expression plasmid carrying plasminogen cDNA

A plasminogen cDNA clone is obtained from a human liver cDNA library after screening with an oligonucleotide probe of sequence:

5′ CC CCT CAC ACA CAT AAC AGG 3′

corresponding to nucleotides 49 to 68 of the sequence described in Malinowski et al Biochemistry 23, p4243 (1984).

A cDNA clone encoding plasminogen and associated 5' and 3' untranslated regions is subcloned into the vector pTRE12 (EP-A-0201153) between the HindIII and BglII sites to create plasmid pTRH5 (Fig.1).

### 1.2. Construction of a hybrid plasminogen: t-PA cDNA molecule

The plasminogen cDNA has two BstXI recognition sites one in the 'A-chain' (at $1209^P$) and one in the 'B-chain' (at $1840^P$).

pTRH5 is cut with BstXI and BglII which gives three fragments:-

(i) 6.4kb BglII (located at 3' end of cDNA)-BstXI ($1209^P$), 'fragment A', containing the vector and approximately 1.15kb of the plasminogen 'A-chain' DNA.

(ii) 630bp BstXI($1209^P$-$1840^P$) , 'fragment B',carrying the remaining part of the plasminogen 'A-chain' DNA including the protease cleavage site and part of the plasminogen 'B-chain'.

(iii) the third fragment of 740bp (BstXI, $1840^P$ to BglII)encoding most of the B-chain is discarded.

Fragments A and B are purified from agarose gels.

A t-PA fragment (C) of 800bp ($1004^t$ to $1805^t$) which encodes the B-chain and 3 amino-acids of the A-chain is isolated from plasmid pTRE24 (EP-A-0201153) by digestion with DdeI and XhoII followed by agarose gel electrophoresis.

To join up the BstXI($906^P$) end with the DdeI ($1004^t$) end, two synthetic oligonucleotides of the following sequences are used:-

3) 5' TGAAGATCTAAGCTTGAAT 3'
4) 5' AAGCTTAGATCT 3'

These oligonucleotides, when combined (fragment D) create restriction sites for HindIII and BglII:

```
        AAGCTTAGATCT
    TAAGTTCGAATCTAGAAGT
    /        |        |        \
Cohesive end X  HindIII  BglII Cohesive end Y
```

where cohesive end X is complementary to that of Bst XI ($1840^P$) in plasminogen cDNA and cohesive end Y is complementary to that of DdeI ($1004^t$) of t-PA cDNA. Fragments A, B, C and D are ligated and transformed into E. coli HB101. Ampicillin resistant colonies are recovered.

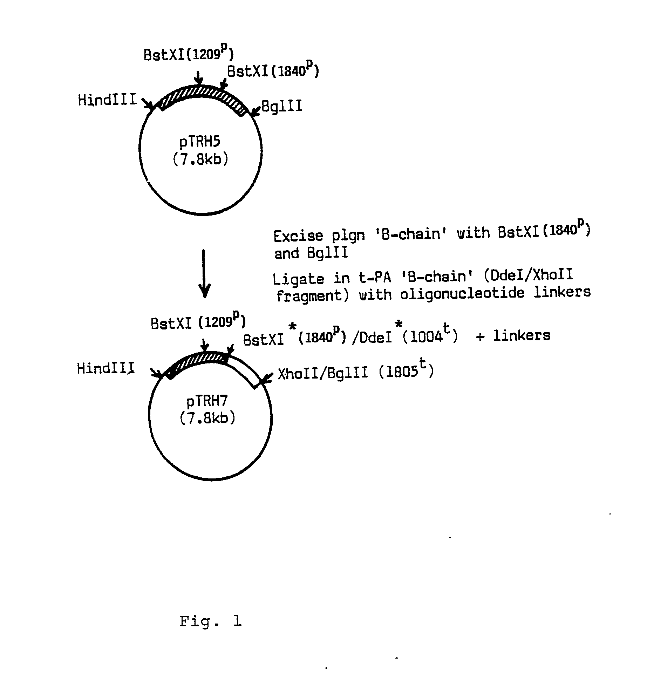

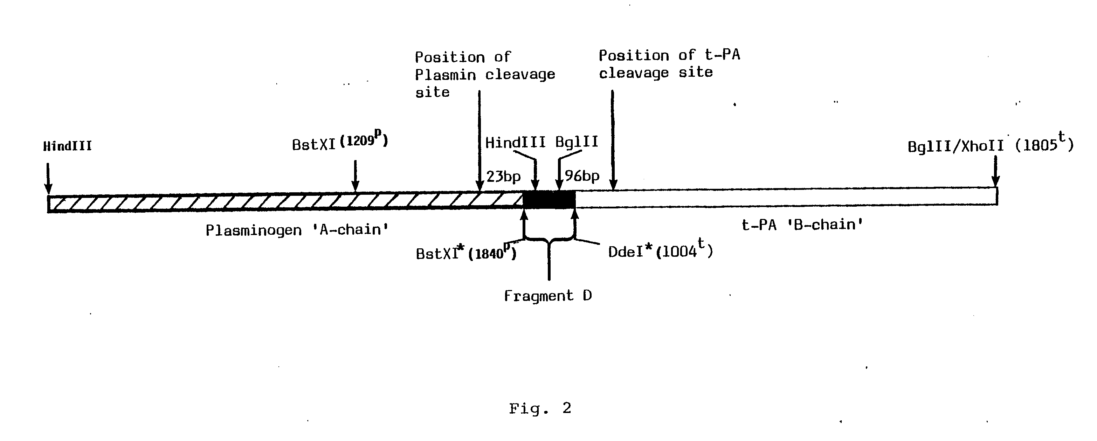

The structure of the hybrid plasmid (pTRH7) isolated from one of these is analysed by digests using the following restriction enzymes: (i) BstXI, unique to the plasminogen 'A-chain' (this enzyme is also diagnostic for the hybrid in that the second BstXI site is destroyed during its construction), (ii) SstI, unique to the t-PA 'B-chain' and (iii) BglII and HindIII, (sites for BglII and HindIII are introduced by fragment D) (Fig.1). The structure of the hybrid gene is believed to be as shown in Fig. 2 and the predicted nucleotide and amino-acid sequences at the plasminogen/t-PA junction are as follows:

```
pro   his   ser   ser*  leu*  asp*  leu*  gln
CCA   CAT   TCA   AGC   TTA   GAT   CTT   CAG
GGT   GTA   AGT   TCG   AAT   CTA   GAA   GTC
```

fragment D

<----plasminogen cleavage          t-PA cleavage site----->
   site 8 amino acids              2 amino acids

Amino acids marked * are added by the incorporation of fragment D. Amino acids to the left of these are plasminogen derived and those to the right are from t-PA.

Example 2

Expression of plasminogen: t-PA hybrid cDNA into fibrinolytically-active protein from L929 cells

A) Fibrin/agar overlay

Expression of pTRH7 from transfected L929 cells (24 hours post transfection) is detected using the fibrin/agarose overlay technique as described in the Methods section. Lytic zones are obtained from pTRH7 but not from cells transfected with the empty parent vector pTRE12 (described in EP-A-0201153).

B) Zymography

The product of the transient expression of pTRH7 is analysed by fibrin zymography as described in Dodd et al., (1986), Thrombosis and Haemostasis 55 (1); 94. The apparent molecular weight is consistent with the predicted structure.

Example 3

Expression of hybrid plasminogen activator from HeLa cells

Cell preparation: HeLa cells were trypsinised and plated out at a density of $1.4 \times 10^4$ cells per $cm^2$ in 30ml (per $175cm^2$ flask) growth medium (this is: Hepes buffered RPM1 1640 medium (041-2400) containing, 10% Serum (021-6010), 1% stock solution of penicillin/streptomycin (043-5070), 2% sodium bicarbonate solution (043-5080), 1% stock Glutamine (043-5030); Gibco, Paisley, Scotland) at 37°C in a humidified incubator in an atmosphere of 5% $CO_2$/95% air. After 72h an extra 25ml of growth medium was added and the cells were used for DNA transfection 24h later.

Transfection procedure: Transfection of plasmid pTRH7 (in Eagles MEM) used calcium coprecipitation as described in 'DNA Cloning' Ed. D.M. Glover, IRL Press, 1985 (Chap. 15, C. Gorman). Glycerol shock and 5mM butyrate treatment were used. Plasminogen activator secreted by transfected cells was harvested in RPMI 1640 medium (as above) but serum-free, containing 4% soybean peptone (Sigma) for 24h. 250ml harvest medium was diluted with 250ml phosphate-buffered saline (Dulbecco's A)/0.1.% Tween 80 (PBS/TW) and was purified using the same method as that described for TRBM6 cell-derived t-PA (Dodd et al (1986) FEBS Lett. 209 13-17). Briefly, the diluted harvest medium was chromatographed on a column (i.d., 32mm; h, 43mm) of Zinc chelate Sepharose CL4B. The hybrid plasminogen activator was dissociated using 50mM imidazole and was passed directly onto a column (i.d., 15mm; h, 28mm) of lysine Sepharose 4B. Purified hybrid plasminogen activator was dissociated from this column using a 0.5M L-arginine-containing buffer.

Two batches of hybrid plasminogen activator were pooled and were concentrated using stirred cell ultrafiltration (YM 10 membrane, Amicon) to approx. 1.5ml and buffer-exchanged using Sephadex G25 (PD10, Pharmacia) into 0.05M phosphate/0.1M NaCl/0.01% Tween 80/10mg ml$^{-1}$ mannitol/1mM EACA pH 7.4 (PST/ME) (2.2ml).

Analysis of the product by SDS PAGE followed by fibrin zymography (Dodd et al, (1986) Thrombos. Haemostas. 55 94-97) showed a major fibrinolytically active species at apparent M$_r$ approx. 100 000, noticeably larger than the lys$_{77}$plasmin A/t-PA B hybrid described in E.P.A. 0155387. Fibrin plate assay with reference to the 1st International Standard for t-PA, Lot 83/517 showed that the product contained 4800 IU.

Sephadex and Sepharose are trade marks.

## Example 4

### Synthesis and characterisation of N,N-dimethyl-4-aminobenzoyl hybrid plasminogen activator

Purified and buffer-exchanged hybrid plasminogen activator from Example 3 (2.2ml, 0.44nmoles) was reversibly blocked at its active centre by gradual addition of 4'-amidinophenyl-N,N-dimethyl-4-aminobenzoate.HCl (AP-DAB)(1mM) at 25°C. The final AP-DAB concentration represented a 48-fold molar excess over the concentration of hybrid plasminogen activator. At 105 mins, 90% inhibition of S2288 amidolytic activity (Dodd et al, (1986) Thrombos. Haemostas. 55 94-97) was detected. Free AP-DAB was removed by buffer-exchange on Sephadex G25 (PD10) in PST/ME. The DAB hybrid plasminogen activator product (3.0ml) was stored at -40°C.

An aliquot of the product was diluted with an equal volume of 0.1M Tris/0.15M NaCl/20%(v/v)-glycerol/0.01% Tween 80 pH 7.4 and incubated at 37°C. Deacylation was monitored by measuring the return of S2288 activity. The deacylation rate constant under the experimental conditions was $2.0 \times 10^{-4} sec^{-1}$.

In the figures:

Figure 1 - Construction of plasmid pTRH7

▨ - plasminogen coding sequences
▢ - t-PA coding sequences
* - restriction site destroyed during construction.

Figure 2 - Expected structure of hybrid gene (from plasmid pTRH7) encloding hybrid plasminogen activator

▨ - plasminogen coding DNA
■ - fragment D
▢ - t-PA coding DNA
* - restriction site destroyed during construction.

## Claims

1. A fibrinolytically active hybrid enzyme which comprises the A chain of plasmin linked to the B chain of a plasminogen activator selected from tissue-type plasminogen activator and urokinase plasminogen activator, characterised in that said chains are linked by peptide bonds via an amino acid sequence comprising the cleavage site of plasminogen.

2. A hybrid enzyme according to claim 1 wherein said amino acid linking sequence comprises residues 562 to 572 of plasminogen.

3. A hybrid enzyme according to claim 1 or 2 wherein said plasminogen activator is tissue-type plasminogen activator (t-PA).

4. A hybrid enzyme according to claim 3 wherein said amino acid linking sequence comprises residues 273 to 275 of t-PA.

5. A derivative of the hybrid enzyme according to any preceding claim.

6. A derivative according to claim 5 wherein any catalytic site essential for fibrinolytic activity is blocked by a removable blocking group.

7. A pharmaceutical composition comprising a hybrid enzyme according to claim 1 or derivative thereof in combination with a pharmaceutically acceptable carrier.

8. A hybrid enzyme according to claim 1 or derivative thereof for use in the treatment of thrombotic diseases.

9. A process for preparing a hybrid enzyme according to claim 1 which process comprises expressing DNA encoding said hybrid enzyme in a recombinant host cell and recovering the enzyme product.

10. A DNA polymer comprising a nucleotide sequence that encodes a hybrid enzyme according to claim 1.

BstXI(1209$^p$)

BstXI(1840$^p$)

HindIII    BglII

pTRH5
(7.8kb)

Excise plgn 'B-chain' with BstXI(1840$^p$)
and BglII

Ligate in t-PA 'B-chain' (DdeI/XhoII
fragment) with oligonucleotide linkers

BstXI (1209$^p$)

BstXI$^*$(1840$^p$)/DdeI$^*$(1004$^t$) + linkers

HindIII

XhoII/BglII (1805$^t$)

pTRH7
(7.8kb)

Fig. 1

Fig. 2